# EUROPEAN PATENT SPECIFICATION

(11) **EP 2 870 945 B1**
(45) Date of publication and mention of the grant of the patent: **06.07.2016**
(21) Application number: 13192089.4
(22) Date of filing: 08.11.2013
(51) Int. Cl.: A61F 2/24

(54) **DEPLOYMENT SYSTEM FOR VASCULAR IMPLANTS**
EINSETZSYSTEM FÜR GEFÄßIMPLANTATE
SYSTÈME DE DÉPLOIEMENT POUR IMPLANTS VASCULAIRES

(43) Date of publication of application: 13.05.2015
(73) Proprietor: NVT AG, 5630 Muri AG (CH)
(72) Inventor: Centola, Marcos, CEP 15025010 S J Do Rio Preto Sao Paulo (BR); Kawa, Emilia, 72379 Hechingen (DE)
(74) Representative: Witte, Weller & Partner Patentanwälte mbB

(56) References cited:
- WO-A2-2012/163820
- US-A1- 2009 287 290

## Description

The present invention relates to a deployment system for deploying a vascular implant. The deployment system comprises first tube comprising a lumen and being designed to carry an expandable vascular implant over a distal portion of the first tube in a loaded state, a sheath designed to be disposed over and holding the vascular implant in a compressed state, wherein the sheath is slidable proximally relative to the vascular implant to stepwise release and expand the vascular implant, and an engaging element for engaging with a proximal end of the vascular implant in its loaded state, wherein the engaging element is disposed about and concentrically aligned with the first tube, and wherein the engaging element is non-slidably positioned between the sheath and the first tube distally to the vascular implant in its loaded state.

Various deployment systems for deploying vascular implants are known in the state of the art. Such deployment systems are mandatory for introducing or delivering a vascular implant into a location of a vessel that needs to be treated in order to either ensure the vessel's functioning, such as the transport of blood, or in order to replace it, such as a heart valve, in which case they are also called vascular prostheses.

In the heart, cardiac - or "aortic" - valves maintain the unidirectional flow of blood by opening and closing depending on the difference in pressure on each side. The aortic valve can be affected by a range of diseases and can, therefore, require cardiac valve replacement, which means that a patient's aortic valve is replaced by a different valve. The valve can either become leaky, i.e. regurgitant or insufficient, in which case the aortic valve is incompetent and blood flows passively back to the heart in the wrong direction. Further, the valve can become partially shut, i.e. stenotic, in which case the valve fails to open fully, thereby obstructing blood flow out from the heart. The two conditions frequently co-exist.

There are two basic types of artificial heart valve, mechanical valves and tissue valves. Tissue heart valves are usually made from animal tissues, either animal heart valve tissue or animal pericardial tissue, which are treated to prevent rejection and to prevent calcification. Whereas mechanical valves generally are designed to outlast the patient, they have the drawback that due to their material there is an increased risk of blood clots forming, which may only be prevented by a constant anti-coagulant therapy, which makes the patient more prone to bleeding. Mechanical heart valves are generally composed entirely of synthetic or nonbiological materials, whereas tissue (or bioprosthetic) heart valves are composed of synthetic and biological materials. Bioprosthetic cardiac valves can either represent xenografts, which are taken from different species than the recipient, or homografts, which are donor valves taken from the same species as the recipient.

Since aortic valve replacement traditionally requires median sternotomy and thus open heart surgery, this surgical intervention has a major impact on the patient. Besides the physical stress associated with this operation, there is a risk of death or serious complications from open heart surgery, in particular depending on the health and age of the patient.

However, with the recently developed technique of transcatheter heart valve replacement, where artificial valves are implanted using a catheter, open heart surgery is no longer necessary. With transcatheter valve replacement, the deployment of the implant can either be achieved retrograde, i.e. against normal blood flow, or ante-grade, with blood flow. Generally, the artificial valves, which are introduced via transcatheter surgery comprise expandable stent systems which are introduced into the vessel in a compressed state and which are allowed to expand by removal of compressive structures.

International patent application WO 2008/070797 discloses a system and a method for transapical delivery of an annulus anchored self-expanding valve. The system disclosed therein comprises a catheter assembly having an outer sheath, an elongate pusher tube and a central tube. At the distal end of the central tube an atraumatic tip is provided; in the loaded state, the implant is carried adjacent to the tip on the central tube and compressed by the sheath. Upon retraction of the sheath, the distal end of the implant, i.e. the one that is located nearest to the tip is released and the implant is allowed to expand. Subsequently, the sheath gets completely pulled back to fully release the implant, which subsequently, in case of as self-expanding implant, can fully expand, or which can be expanded by using a balloon.

Similarly, WO 2007/098232 discloses a deployment device for self-expanding prostheses, wherein the device comprises a split sheath by means of which the implant can be deployed in several steps.

Usually, the heart valve is deployed in two steps, whereby first the proximal end - that is usually hold by the tip - has to be deployed, followed by the deployment of the distal end. The precise deployment with the possibility to move the implant once partially deployed still remains a crucial step. In particular, with the prostheses and deployment systems presently used and known in the state of the art, there is the danger that the implant with its proximal end gets trapped upon deployment of the proximal end of the implant due to the deployment system's design of the tip.

As a consequence, trapping of the proximal end of the implant leads to an incorrectly placement of the proximal end and to the situation that an incorrect placement of the implant cannot be corrected, since it cannot be moved within the vessel. In the worst scenario, the implant cannot be deployed at all, nor even be retrieved from the vessel.

WO 2012/163820 A2 discloses a deployment system for deploying expandable cardiac valve prostheses. The system has a first tube and a tip being connected with the tube, and which tube detachably accommodates and holds a distal end of a cardiac valve prosthesis. The system also comprises a biasing element being linked to the tip, and a blocking mechanism in the proximal direction relative to the tip which blocking mechanism limits the movement of an actuating mechanism in the proximal direction relative to the tip.

Against this background it is an object of the invention to provide for an deployment system by means of which an entrapment of the implant can be avoided.

According to the invention, this and other objects are achieved by a deployment system as described in detail in the claims.

Accordingly, the invention concerns a deployment system for deploying a vascular implant, the deployment system comprising a first tube comprising a lumen and being designed to carry an expandable vascular implant over a distal portion of the first tube in a loaded state and comprising a sheath designed to be disposed over and holding the vascular implant in a compressed state, wherein the sheath is slidable proximally relative to the vascular implant to stepwise release and expand the vascular implant; the deployment system further comprises an engaging element for engaging with a distal end of the vascular implant in its loaded state, the engaging element being disposed about and concentrically aligned with the first tube, wherein the engaging element is non-slidably positioned between the sheath and the first tube proximally to the vascular implant in its loaded state; the deployment system according to the invention also comprises a tip, being fixedly connected to the first tube at a distal end of the first tube, wherein the tip comprises a first proximal end and a second distal end, the first proximal end having a port for detachably accommodating and holding a proximal end of the vascular implant in a loaded state, wherein the tip is slidable distally relative to the vascular implant to release the proximal end of the vascular implant form the port; according to the invention, the deployment system further comprises a spring mounted plug element being slidably mounted over the first tube and being designed such that in the vascular implant's loaded state the plug element distally abuts engaging element, and that upon release of the proximal end of the vascular implant from the tip's port the plug element is engagable with and closes the port.

With the deployment system according to the invention, an entrapment of the implant in/with the tip can efficiently be avoided, since the plug element, upon release of the proximal end of the implant, instantly closes the open proximal end of the tip, which, prior to the deployment, held the proximal end of the implant. The closure of the tip is achieved by the plug element being spring-loaded and by the spring effectuating the plug element as soon as the proximal end of the implant gets released from the tip's proximal end.

At the same time, the blood flow during deployment of the implant is guaranteed, since a flaring of the proximal end prior to the correct placement is prevented. Also, a deployment of the distal end of the implant prior to the deployment of the proximal end of the implant, which would obstruct blood flow, is successfully prevented. This is achieved by the tip accommodating the proximal end of the implant, and by the fact that the distal end of the implant remains attached and in a compressed state after the proximal end has been released.

Also, with the deployment according to the invention, a controlled, balloon-like release of the medial portion of the implant is effected, which allows a precise placement, and even a correction, i.e. back- and forth movement, of the implant during deployment. With this balloon-like deployment, flow of the blood past the implant is guaranteed, while at the same time entrapment of the once released proximal end of the implant is prevented.

In the prior art, as well as in the present application, the end of the implant which lies, in its deployed state, closer to the heart, is designated as the proximal end of the implant, whereas the end of the implant which lies farther away from the heart than the other end of the implant is designated as the distal end.

Accordingly, the end of a cardiac valve implant (or "heart valve") comprising a valve, is usually called the "proximal end", whereas the end of the heart valve further free from valve material is called the "distal end".

In contrast, the designation of the ends or end portions of the deployment system as distal and proximal is such that the end closer to the operator is designated as "proximal" proximal and the other end further from the operator is designated "distal". Presently and throughout this application, if the expression "proximal" is used in connection with designating a direction, i.e. the "proximal direction", the direction towards the operator is meant. On the other hand, if the expression "distal" is used to describe a direction, i.e. the "distal direction", the direction leading or pointing away from the operator/surgeon/practitioner is meant. Also, when designating the ends of the parts of the deployment system, the term "proximal end" refers to the end that is nearest to the operator, e.g. the surgeon, and the term "distal end" refers to the end opposite to the proximal end and is nearer to the patient to be treated.

According to a preferred embodiment of the invention the plug element is fixedly connected with a spring element.

Presently, a "spring element" is meant to encompass an elastic element that offers resistance to a compressive force applied axially.

With the plug element being fixedly connected with a spring element, the force resting in the spring is, upon release of the proximal end of the implant from the proximal end of the tip, used to force the plug element towards the now open proximal end of the tip. The plug element, in the loaded state and prior to the release of the proximal end of the implant, lies proximally relative the tip and proximally relative to the implant, i.e. nearer to the person handling the deployment system. Upon release of the proximal end, the plug element is forced - via the spring element - towards the now open port of the tip thus closing it and thus preventing the implant getting entrapped in the tip.

Presently, the expression "port" in view of the tip is meant to designate an open section of the tip, where the proximal end of the implant can be hold within and released from upon deployment of the proximal end. Accordingly, the port "is open" when the proximal end of the implant is released from the tip, and the port is "closed" if it is either holding the proximal end of the implant in the loaded state, or if it is closed by the plug element.

An "engaging element" as it is presently understood in the present invention encompasses every means that is suitable to provide for a temporary engagement or fixation of the distal end of the implant proximal end of the vascular implant in its loaded state, whilst being disposed about and concentrically aligned with the first tube distally from the tip and distally from the plug element.

According to a preferred embodiment, the spring element comprises a compression spring, wherein it is further preferred if it comprises a proximal open pitch section and a distal closed pitch section.

A compression spring is an open-coil helical spring that offers resistance to a compressive force applied axially. Thus, and according to the invention, the compression spring element is, when the implant is loaded on the deployment system, compressed in proximal direction, i.e. towards the person handling the deployment system, and upon release of the proximal end of the implant, the spring element decompresses and forces the plug element in the distal direction, i.e. towards the tip thus closing its open port the proximal portion of the implant was stored.

As it is generally known in the art, a "pitch" of a spring is the distance from the center of one coil to the center of the adjacent coil like threads.

Thus, according to a preferred embodiment, and as mentioned above, the spring element/the compression spring comprises a proximal open pitch section, meaning that the coils of this section do not touch each other, i.e. the distances between the coils are not closed, and a distal closed pitch section, where there is no distance between the coils, and the coils are closed. In this embodiment, the section with the open pitches serves as the part of the spring element forcing the plug element towards the tip upon release of the proximal end of the implant, and the section with the closed pitches serves as attachment for the plug element, while simultaneously protecting the implant since no open coils are exposed in this section when the plug is forced through the expanded implant leaving no entrapment means.

Preferably, the closed pitch section has a length corresponding to about the length of the implant.

According to a preferred embodiment, the plug element comprises a compressible material, preferably coated silicone. This embodiment has the advantage that the plug element is compressible when the implant is loaded on the deployment system; further, with coating, a smooth sliding of the plug element into the open port of the tip can be achieved.

In a preferred embodiment of the deployment system according to the invention, the engaging element is fixedly supported on a shaft being disposed about and concentrically aligned with the first tube, wherein the shaft is non-slidably positioned between the sheath and the first tube.

In this embodiment, the engaging element is mounted on a shaft, which, in the deployment system's overall assembly, is positioned between the sheath and the first tube.

The shaft according to the invention represents a pusher or a "back-up" shaft that holds the implant in position when the sheath is being pulled back from the implant, i.e. in the proximal direction. The shaft according to the invention comprises, at its distal end, the engaging element which represents means for connecting and temporarily holding the distal end of the implant to the shaft. Upon complete removal of the sheath compressing the implant, the distal end of the implant is released from the shaft.

Preferably, the engaging element of the shaft is a circumferential, ring-like protrusion located in the distal portion of the shaft, preferably in a certain distance from the very distal end of the shaft. The distal end of the implant can be placed over the protrusion and the sheath compressing the implant may be placed over the thus temporarily hooked end of the implant, thus preventing the end of the implant to move with the sheath upon its removal.

According to another preferred embodiment, the deployment system according to the invention further comprises at least one of the following: a first actuating mechanism being linked to the sheath and being slidable in a proximal direction for stepwise retracting the sheath; a second actuating mechanism for moving the tip in the distal direction, thus releasing the proximal end of the implant; a biasing element provided in a proximal portion of the first tube and being directly linked to the tip, and

and/or a blocking mechanism being designed such, that a movement of the first actuating mechanism in the proximal direction relative to the tip is limitable to a maximum path of travel by the blocking mechanism.

With the above additional features, alone or in combination, an effective, smooth and secure deployment of the implant, in particular of a heart valve can be accomplished.

With the deployment system according to the invention, the blood flow during deployment of the implant is guaranteed, since a flaring of the proximal end prior to the correct placement is prevented. Also, a deployment of the distal end of the implant prior to the deployment of the proximal end of the implant, which would obstruct blood flow, is successfully prevented. This is achieved by the tip accommodating the proximal end of the implant, and by the fact that the distal end of the implant remains attached and in a compressed state after the proximal end has been released.

Also, this is achieved through the deployment of the medial portion of the implant, i.e. through the controlled, balloon-like release of the medial portion, which allows a precise placement, and even a correction, i.e. back- and forth movement, of the implant during deployment. With this balloon-like deployment, flow of the blood past the implant is guaranteed, which, with the deployment system of the state of the art, is, due to the flared end of the implant, prohibited.

With the blocking mechanism as it may be comprised in the deployment system according to the invention, this balloon-like deployment can be achieved, since the blocking mechanism prevents that the proximal end is deployed before the implant is placed on its precise location. A partial release of the implant is, however, needed, since only with the partial, balloon-like expansion of the implant the dimension and placement of it and its intended location can be assessed.

With the second actuating mechanism the tip can be moved in the distal direction, thus releasing the distal end of the implant. The operator may actuate this second actuating mechanism as soon as the intended site of deployment is reached. Upon actuating the second actuating mechanism, the tip can be moved in the distal direction, thus releasing the proximal end of the implant.

According to the invention, and as described above, upon release of the proximal end of the implant, the load on the spring element is released thus forcing the plug element towards and into the port of the tip.

Preferably, the tip is engaged with the biasing element, and the tip is, due to the biasing element, released in a resilient or spring-loaded manner, i.e. the tip is, by means of the then stress-loaded spring, briefly pushed in the distal direction and subsequently moved back - due to the load being relieved from the spring - and into the thus expanded implant.

In a refinement of the present invention, the blocking mechanism comprises a punch element, spring-loaded wing elements, and an abutment element,

Presently, the term resilient or "spring-loaded wing element" means any oblong element that aligns with a longish basis and has one of its end attached to the basis, wherein its other end is freely moveable, preferably under a force or pressure-load, away from the basis wherein the other end remains attached to the basis.

Further, the expression "punch element" presently means any element that is suited and designed to press or force apart resilient elements, when moved in between the resilient elements.

The expression "abutment element" presently means any element suitable and designed to provide for a mechanical stop via a face or a wall when another moveable element is moved towards that abutment element.

According to an embodiment of the present invention, the wing-elements are designed such, that their ends that are attached to the basis are located proximally, and that their freely moveable ends are positioned distally.

According to another embodiment of the present invention, the biasing element is a spring, compression spring, or tension spring.

Furthermore, the biasing element is, via the punch, designed to engage with the blocking mechanism.

With this embodiment, the biasing element, upon actuating the second actuating mechanism, is moved in distal direction to release the proximal end of the implant, and subsequently moved into the implant, thus releasing the resilient pressure on the blocking mechanism. Upon the release of the blocking mechanism, the first actuating mechanism may be actuated again and moved further in the proximal direction to fully deploy the implant.

In a preferred embodiment, the punch element of the blocking mechanism is forceable in between the spring-loaded wing elements, thus forcing the freely moveable ends of the wing elements apart, and into a stressed position, with the freely moveable ends of the wings abutting against the abutment element positioned distally from the wing elements, thus blocking the actuating mechanism's path of travel in the proximal direction.

With this feature, it is achieved that the first actuating mechanism cannot move unlimited in the proximal direction, thus preventing that the implant gets fully deployed prior to its correct positioning. The maximal path of travel the first actuating mechanism may travel until it gets blocked by the blocking mechanism corresponds to the maximal length of the implant' balloon-like expansion without one of its proximal or distal ends being released.

In other words, in yet another embodiment of the present invention, the first actuating mechanism and the abutment element are, prior to the deployment of the self-expanding cardiac valve implant, spaced apart from one another in a distance corresponding to a first retraction length of the sheath, the first retraction length of the sheath being such that a medial portion of the implant is released and the proximal and the distal ends of the implant are still covered by the tip and the sheath, respectively.

According to another embodiment, the first actuating mechanism comprises an operator engagable handle.

With "operator engagable handle", a handle is meant which can be directly gripped and manipulated/actuated by the operator, and may include, e.g., a resilient or spring-loaded lever. The lever is connected via a hinge at one of its ends to the body of the actuating mechanism, whereby its other end is freely moveable. In its relaxed state, the spring-load is forcing the freely moveable end angularly away from the basis, whilst its other end remains attached to the basis. The lever or handle can be pressed towards the longitudinal axis against the spring-exerted forces, thus actuating the actuating mechanism.

According to another embodiment of the invention, the deployment system further comprises a guidewire lumen, which is preferably provided within the first tube.

With the guidewire lumen provided in the first tube, the deployment system can be guided over a guide wire, which has firstly been introduced into the vessel of a patient's body, thus facilitating the introduction of the deployment system.

According to yet another embodiment, the deployment system further comprises a tubular knurled or toothed rod disposed about and concentrically aligned with the proximal portion of the sheath. The lever of the first actuating mechanism engages with the tubular rod, thus allowing for a stepwise retraction of the sheath upon actuation of the lever/first actuating mechanism.

In a preferred embodiment, the rod comprises at least one fixation element by means of which, preferably with its proximal end, it can be releasably attached within the abutment element. Upon engagement with the abutment element, the tubular rod cannot be moved relative to the abutment element and vice versa.

Preferably, the proximal end of the rod comprises two at least partially resilient fixation elements pointing outward from a longitudinal axis of the rod and engaging with the abutment element. In this connection the expression "resilient" shall mean, that the fixation elements can be transferred from a pressure-free/unloaded position where it/they is/are engaging the abutment element/the housing of the abutment element and where it/they is/are pointing away from the longitudinal axis of the rod, to a pressure-loaded position, where they are moved towards the axis of the rod, thus detaching from the engagement with the abutment element. Thus, the fixation elements represent protrusions, projecting away and outwardly from a longitudinal axis of the tubular rod. Via this/these fixation element(s), the rod is attached to the abutment element, and, as a consequence, this/these stop element(s) are blocking the abutment element to move in proximal direction. Thus, the attachment of the rod within the abutment element represents an additional safety means, preventing a premature release of the implant.

Preferably, there are provided two fixation elements which are positioned opposite from one another on the circumference of the rod.

With this measure, an outer housing for the sheath is provided accommodating the proximal end of the sheath therein; the actuating mechanism, being connected with the proximal end of the sheath can, upon its actuation, slide over the second tube.

The present invention also relates to a deployment system as described above, further comprising a vascular implant, in particular a cardiac valve implant.

Also presently disclosed is a method for releasing a cardiac valve implant loaded on a deployment system, the method comprising the steps of: a) providing a deployment system according to the invention, comprising an expandable vascular implant, b) actuating the first actuating mechanism of the deployment system, and, thus, stepwise retracting the sheath and simultaneously moving the actuating means in the proximal direction until the actuating mechanism abuts the blocking mechanism, whereupon the punch element is forced in between the resilient wing elements, thus forcing them apart and blocking further movement of the actuating mechanism and the retraction of the sheath, thus, c) releasing a medial portion of the implant, whereby the proximal end of the implant remains fixed within the tip, and the distal end of the implant remains fixed in the distal end of the shaft, thereby permitting a balloon-like expansion of the implant, d) moving the tip and, thus, releasing the proximal end of the implant, whereby the pressure on the biasing element is relieved, thus withdrawing punch element from in between the resilient wing elements, thus releasing the resilient wing elements into an unstressed position, and simultaneously forcing the plug element into the port of the tip, and e) fully retracting the sheath and fully deploying and releasing the expandable implant.

With this method, a precise placement of the implant is possible. Also, employing this method allows the practitioner or surgeon to carefully place and even replace the implant, without being under time pressure for timely positioning the implant in order not to obstruct blood flow. Also, with the method according to the invention, an entrapment of the tip with the implant is successfully avoided, since the plug element provides for a closed structure of the proximal end of the tip. Also, with the method according to the invention, a balloon-like intermediate step of deploying the implant is generated, and flow of blood past the implant is guaranteed, thus providing time for its deliberate deployment.

In case an cardiac or heart valve is to be deployed, the method may further comprise, after the providing-step, the step of positioning the deployment system such, that the implant is in the area of the cardiac valve to be replaced by the implant.

Distally moving the tip is preferably achieved by actuating a second actuating mechanism.

It is understood that the features described hereinabove and those still to be described below fall within the scope of the present invention not only in the respectively specified combinations, but also in different combinations or on their own.

Further features follow from the description and the preferred embodiments.

Preferred embodiments are shown in the Figures and are described in further detail herein below.

In the figures:
- Fig. 1: shows, in longitudinal section, an embodiment of a deployment system according to the invention, not drawn to scale, in an unloaded state and with retracted sheath, i.e. without an implant being loaded on the deployment system;
- Fig. 2: shows the deployment system of Fig. 1, with an implant partially deployed, in an intermediate step of the deployment, not drawn to scale (2A); an enlarged detail of an proximal portion of the deployment system shown in the position as depicted in Fig. 2A (B); and
- Fig. 3: shows the deployment system of Fig. 1, with the implant deployed at its proximal end.

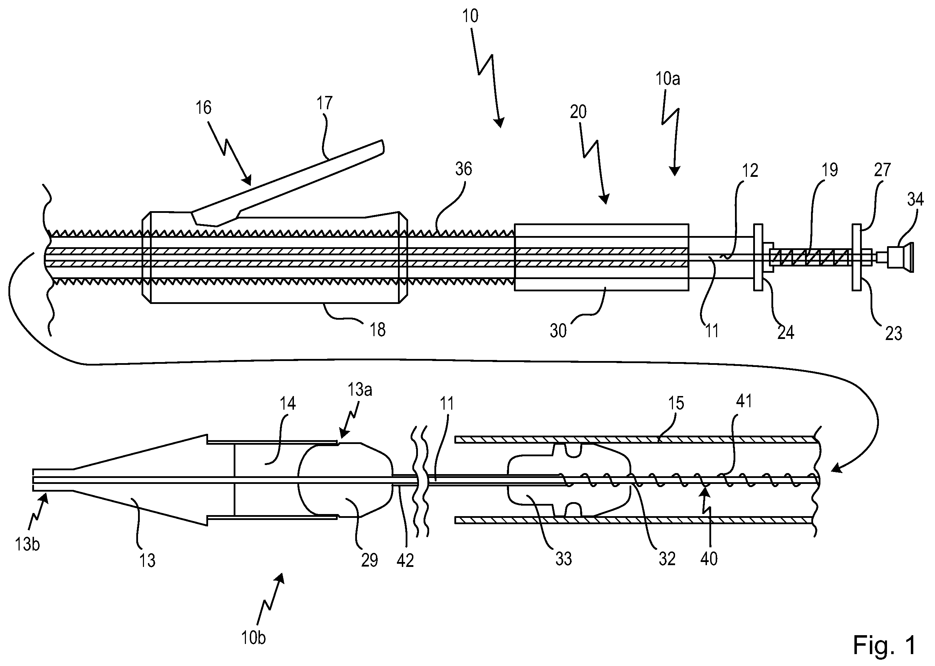

In Fig. 1, an embodiment of the deployment system according to the invention is generally designated with 10. The deployment system 10, not drawn to scale and showing a proximal portion 10a and a distal portion 10b of the deployment system, comprises a first tube 11, having a lumen 12 designed for accommodating a guidewire (not shown).

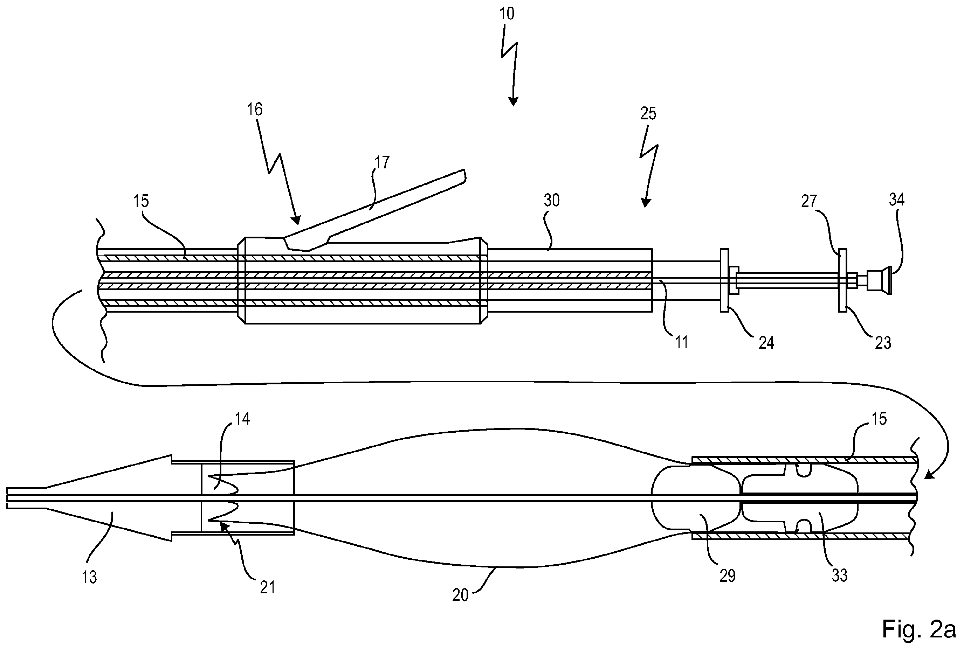

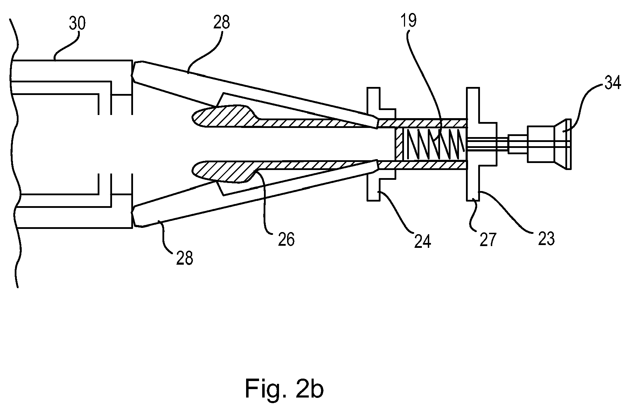

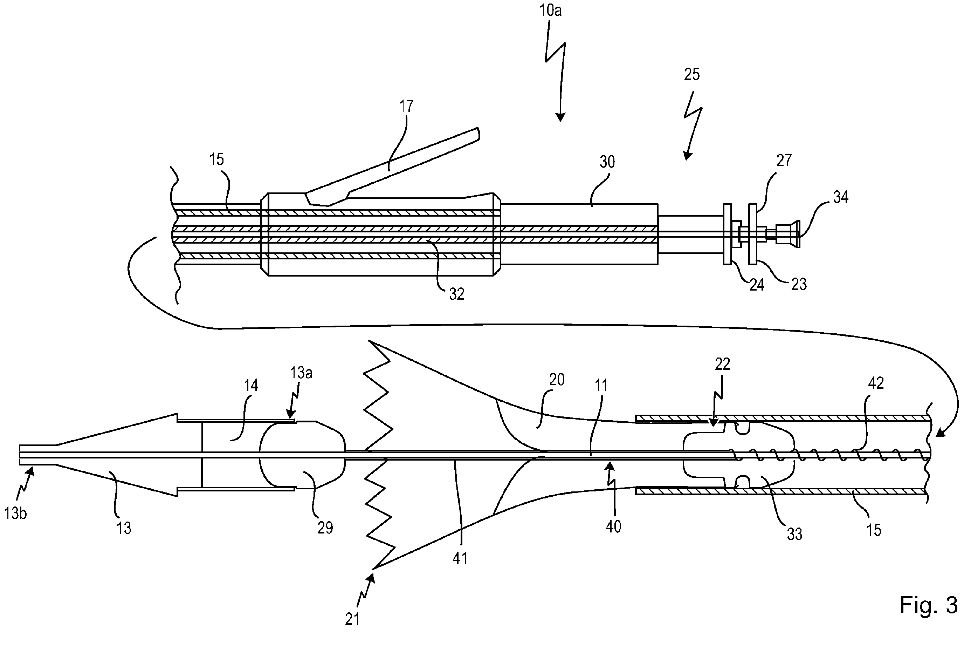

In Fig. 1, the deployment system 10 also comprises an outer sheath 15, which is partially retracted. The tube 11 has, on its very distal end, a tip 13 representing an atraumatic tip, having, on its side facing towards the proximal direction, i.e. the tip's 13 proximal end 13a, means or a port 14 for accommodating and detachably holding a proximal end of an implant (see fig. 2 and 3). The tip 13 comprises also a distal end 13b facing towards the distal direction, i.e. away from the operator.

In the state as shown in Fig. 1, which represents the state prior to loading an implant onto the deployment system or the state after deployment of an implant, a plug element 29 is present in the port 14 of the tip's 13 proximal end 13a, thus closing it. The plug element 29 is spring-loaded and mounted onto a spring element 40, which comprises a proximal open pitch section 41 and a distal closed pitch section 42; as can be seen from Fig. 1, the open pitch section 41 lies within or beneath the portion of the deployment system covered by a shaft 32, whilst the closed pitch section, upon retracting of the sheath 15 and release of the implant is exposed.

Further, in the deployment system 10 according to the invention, engaging element or means 33 is shown, which serve as engaging means for the distal end of an implant in its loaded state. The engaging element 33 is, in the exemplary embodiment depicted in the figures, a circumferential ring-like protrusion placed about the distal portion of a shaft 32 in a certain distance from the very distal end of the shaft 32. The distal end 22 of the implant 20 can be detachably hooked onto this protrusion/engaging element 33.

The outer sheath 15 is intended to hold and compress an implant in a compressed state when loaded on the first tube 11. The outer sheath 15 is slidable/retractable in proximal direction, i.e. towards the operator, in order release the implant and to allow its expansion. The outer sheath 15 is linked to a first actuating mechanism 16, the actuating mechanism 16 being slidable in a proximal direction for stepwise retracting the sheath 15.

Also shown in fig. 1 is actuating mechanism 16 which comprises a lever 17, linked to a tube-like grip 18, which is disposed about the outer sheath 15. Internally of the grip 18, the outer sheath 15 is attached within the grip 18. The lever 17 is, by means of a spring-loaded mechanism (not shown) attached, via one of its ends 17a and via a hinge (not shown) to the grip 18. Its other end 17b is freely moveable from a closed, stress-loaded position, where the lever 17 is approached to the grip 18, to an open, stress-relieved position, where the spring/hinge keeps and forces the lever 17 angularly away from the grip 18. By stepwise actuating the actuating mechanism 16, i.e. by repeatedly pressing down the lever 17, the sheath 15, connected with the grip 18, is stepwise retracted.

As can be seen in Fig. 1, the deployment system 10 further comprises a biasing element 19 provided in the proximal portion 10A of deployment system 10 and being linked to the tip 13. The biasing element 19 is securely placed and provided between two holding elements, a proximal holding element 23 and a distal holding element 24, which are, in a defined distance from one another, placed over the first tube 11. Holding element 23 represents a second actuating mechanism 27, which will be explained further below.

The deployment system 10, in addition, comprises a blocking mechanism 25, having a punch element 26, resilient or spring-loaded wing elements 28, and an abutment element 30. The distal holding element 24 is engaged with the punch element 26 and the resilient wing elements 28.

As mentioned briefly above, shaft 32 which is disposed about and concentrically aligned with the first tube 11, and is non-slidably positioned between the sheath 15 and the first tube 11, comprises the engaging element or connecting means 33 for engaging with the distal end of the implant. The engaging element is, in the exemplary embodiment depicted in the figures, a circumferential ring-like protrusion placed about the distal portion of the shaft 32 in a certain distance from the very distal end of the shaft 32. The distal end 22 of the implant 20 can be detachably hooked onto this protrusion/connecting means 33.

Also provided in the deployment system 10 according to the invention is a second actuating means 27, representing, in the exemplary embodiment shown in the figures, a knob, directly linked with the tip and being located at the proximal end of the first tube. The knob may be pushed in the proximal direction to actuate the tip 13, whereby the tip 13 gets pushed first in the distal direction to release the proximal end 21 of the implant 20, and subsequently gets drawn back, i.e. in the proximal direction and into the then open and expanded proximal end 21 of the implant 20, which movement of the tip 13 is effected by the biasing element 19.

In the Figures, reference sign 34 designates the adaptor for the guide wire lumen.

References sign 36 designates a knurled or toothed tubular rod, which can be, as shown in fig. 1, positioned concentrically about the sheath and which interoperates with the actuating mechanism 16, in particular with the lever 17, such that a stepwise retraction of the sheath is allowed upon actuating the actuating mechanism 16. The lever 17 is, via its end 17a engaging with the knurled rod. The rod 36 is, with its proximal end attached within the abutment element 30. For clarity reasons, the rod 36 is only shown in fig. 1A, and not in the figures 2 and 3.

In Figures 2 and 3, deployment steps for deploying - at least partially - an exemplary implant, i.e. a cardiac valve implant, are shown:

The blocking mechanism 25 is designed such, that a movement of the first actuating mechanism 16 in the proximal direction relative to the tip 13 is limitable to a maximum path of travel by the blocking mechanism 25. This is achieved by the interaction of the first actuating mechanism 16 with the blocking mechanism 25 and is shown in Fig. 2: Upon actuation of the first actuating mechanism 16, i.e. by pressing down the lever 17 towards the grip 18, the first actuating mechanism 16 is stepwise moved in proximal direction; the movement of the actuating mechanism 16 in proximal direction is then stopped when it abuts the abutment element 30, which serves as a safety locker.

This is also due to the fact that the biasing element 19 interacts with the punch element 26 and exerts pressure on the punch element 26, since the biasing element 19 gets stress-loaded through the abutment of the actuating mechanism 16 on the abutment element 30, which can be seen in Fig. 2B.

As can also be taken from Fig. 2A, the implant 20 gets, upon stepwise movement of the actuating mechanism in the proximal direction, stepwise released in a medial portion, whilst its proximal end 21 and its distal end 22 remain affixed to the tip's 13 port 14 and the engaging element 33 at the distal end of the shaft 32, respectively. In this way, the implant 20 expands in a balloon-like manner, facilitating flow of the blood past the implant 20, thus not obstructing the normal blood flow through the heart.

Further, with the implant 20 not completely expanded and still fixed with its proximal 21 and its distal 22 end, a precise positioning of the implant 20 is still possible, i.e. movement and shifting of the balloon-like expanded implant 20 can be performed to correctly place it in the vessel. Also, if a reloading of the implant 20 into the deployment system 10 should be necessary or wanted, the sheath 15 may be simply repositioned over the medial portion of the implant 20, by moving the first actuating mechanism 16 in the distal direction.

As can be also taken from fig. 2a, the plug element 29 is, due to the implant loaded on the deployment system and, thus, due to the implant's load on the spring element 40, forced against the load of the spring towards the proximal direction and next to the engaging element 33, whereby the closed pitch section portion 42 of the spring element 40 is also positioned beneath the shaft 32/sheath 15, whilst the open pitch section 41 is, as a consequence, positioned even farther towards the proximal direction.

In a next deployment stage, which is depicted in Fig. 3, the knob, i.e. the second actuating mechanism 27 is actuated, thereby moving the tip 13, in a spring-loaded manner, in the distal direction to release the proximal end 21 of the implant 20. Immediately after release of the proximal end 21 of the implant 20, the plug element 29 is - via the spring element 40 - forced towards the tip 13 and into the tip's 13 port 14, thus closing it. With the load exerted by the implant 20 released, the plug element 29 is instantly moved into the opening of the tip and prevents the tip 13 from entrapment with the implant. This is crucial, since the tip 13 gets subsequently moved back into the then open and expanded proximal end 21 of implant 20. The implant 20 is, upon release of its proximal end 21, which comprises the valve, fully functional and starts to work. In that way, blood flow gets, at no point of the deployment, obstructed by the deployment or introduction of the implant 20.

It can be seen that the spring element's 40 open pitch section 41 is moved towards the distal direction, too.

With the tip 13 moving inside the implant 20, the punch element 26 is shifted into its most proximal position, thereby moving out from in between the spring-loaded wing elements 28. As a consequence, the spring-loaded wing elements 28 are centred back towards their basis, thus no longer blocking the abutment element 30.

In case the tip 13 should nevertheless jam with or in the implant 20 and should, therefore, not be moved into/inside the implant 20, the punch element 26 is not shifted into its most proximal position, thereby still forcing the wing elements 28 apart and against the abutment element 30, thus still blocking the abutment element 30. In that way, the deployment system 10 according to the invention provides for an additional safety mechanism, which guarantees that the distal end 22 of the implant 20 cannot be deployed before the end 21 of the implant 20 has been correctly released. To release the eventually jammed tip 13, the knob or second actuating mechanism 27 has simply to be pushed another time taking care that the tip 13 does not get jammed again.

As a next deployment stage, and in case the tip 13 is in the correct position, the abutment element 30, together with and driven by the first actuating mechanism 16 abutting against the abutment element 30, can be moved in the proximal direction, whereby the sheath 15 gets completely retracted, releasing and freeing the distal end 22 of the implant 20 (not shown).

The method according to the invention by means of which an implant 20 can be deployed in, e.g., a heart of a patient in need thereof, which may be a human or any other mammal, comprises the deployment steps described above in connection with the description of the figures.

Generally, as a first step, a guidewire is introduced via blood vessels and advanced into the heart. Subsequently, the deployment system 10 the elements of which are carrying and holding the implant 20 in a compressed state is inserted over the guidewire, whereby its advancement is being controlled by, e.g., X-ray or other radiographic means. The very proximal end 21 of the implant 20 is via the tip's 13 port 14 detachably attached to the tip 13, the very distal end 22 is detachably fixed to engaging means 33 in the distal portion of shaft 32, and the implant 20 is overall covered by the sheath 15, which compresses and restrains implant 20.

As soon as the operator/practitioner/surgeon is of the opinion that the implant 20 is correctly placed, he actuates and moves the first actuating mechanism 16 in the proximal direction, thus stepwise retracting the sheath 15, whereby the medial portion of the implant 20 is allowed to expand. In a second deployment stage, the first actuating mechanism 16 abuts the abutment element 30, and the biasing element 19, which is connected with the tip 13, forces the punch element 26 in between the wing elements 28, forcing them apart and blocking any further proximal movement of the first actuating mechanism 16 and abutment element 30. The implant 20 may now be precisely placed (or even reloaded, if necessary) by moving the deployment system 10, and thereby the implant 20, back and forth.

In a third deployment stage, the proximal end 21 of the implant 20 is released by actuating the second actuating mechanism 27/knob, which is directly connected with the tip 13. In doing so, the tip 13 springs in distal direction and thus releases the proximal end 21 of the implant 20, whereupon the plug element 29 is moved towards the tip and into the tip's 13 port 14 thus closing it. The tip 13 can then be moved back in proximal direction and into the opened and expanded implant 20.

With the tip 13 now being located inside the implant 20, the punch element 26 gets moved in its most proximal position, thus moving out from between the forced-apart wing elements 28, whereby the wing elements 28 get - due to their spring load - forced back and aligned towards their base. As a consequence, the abutment element 30 as well as the first actuating mechanism 16 are not blocked anymore and may be moved in proximal direction to retract the sheath 15 and to fully release the implant 20.

The safety measures that are provided with the deployment system 10 according to the invention are, thus, a guarantee that the proximal end 21 of the implant 20 gets deployed before the distal end 22 of the implant 20; that the implant end does not flare upon deployment and, thus, does not obstruct blood flow, allowing a more precise and controllable placement of the implant 20; and that back and forth movements of the implant 20 are possible, crossing the native valve, due to the balloon-like shape of the partially deployed implant 20.

Furthermore, the implant 20 may be reloaded when in the balloon-like stage, if necessary. Also, with the movement of the tip 13 closed with the plug element 29 inside the implant 20, the tip 13 does not drag or entrap with - and potentially damage the valve - the implant, but rather allows for a smooth retraction and removal of the deployment system 10, including the tip 13.

In addition, the deployment step of forming a balloon-like expanded implant 20 allows for a better orientation of the implant 20 and for a more precise placement in the heart.

The implant that is used with the deployment system according to the invention comprises a self-expandable material, preferably Nitinol or any other metal with shape-memory characteristics. The valve comprised in the implant in case a heart valve implant is implanted, may be a donor valve, e.g. a valve from a mammal, or an artificial valve, as outlined in the outset.

While various embodiments of the invention have been described, it will be apparent to those of ordinary skill in the art that many more embodiments and implementations are possible within the scope of the invention. Furthermore, although various indications have been given as to the scope of this invention, the invention is not limited to any one of these but may reside in two or more of these combined together. Accordingly, the invention is not restricted except in light of the attached claims and their equivalents.

## Claims

1. Deployment system (10) for deploying a vascular implant, the deployment system comprising:
a first tube (11) comprising a lumen (12) and being designed to carry an expandable vascular implant over a distal portion (11 a) of the first tube (11) in a loaded state,
a sheath (15) designed to be disposed over and holding the vascular implant (20) in a compressed state, wherein the sheath (15) is slidable proximally relative to the vascular implant (20) to stepwise release and expand the vascular implant (20),
an engaging element (33) for engaging with a distal end (22) of the vascular implant in its loaded state, the engaging element being disposed about and concentrically aligned with the first tube (11), wherein the engaging element is non-slidably positioned between the sheath (15) and the first tube (11) proximally to the vascular implant in its loaded state;
a tip (13), being fixedly connected to the first tube (11) at a distal end of the first tube (11), wherein the tip (13) comprises a first proximal end (13a) and a second distal end (13b), the first proximal end (13a) having a port (14) for detachably accommodating and holding a proximal end (21) of the vascular implant (20) in a loaded state, wherein the tip (13) is slidable distally relative to the vascular implant (20) to release the proximal end (21) of the vascular implant (20) form the port (14),
**characterized in that** the deployment system (10) further comprises a spring mounted plug element (29) being slidably mounted over the first tube (11) and being designed such that in the vascular implant's (20) loaded state the plug element (29) distally abuts engaging element (33), and that upon release of the proximal end (21) of the vascular implant from the tip's (13) port (14) the plug element (29) is engagable with and closes the port (14).

2. The deployment system of claim 1, **characterized in that** the plug element is fixedly connected with a spring element (40).

3. The deployment system of claim 2, **characterized in that** the spring element (40) is a compression spring.

4. The deployment system of claim 2 or 3, **characterized in that** the spring element (40) comprises a proximal open pitch section (41) and a distal closed pitch section (42).

5. The deployment system of any of claims 1 to 4, **characterized in that** the plug element (29) comprises a compressible material, preferably coated or uncoated silicone.

6. The deployment system (10) of any of the foregoing claims, **characterized in that** the engaging element (33) is fixedly supported on a shaft (32) being disposed about and concentrically aligned with the first tube (11), wherein the shaft (32) is non-slidably positioned between the sheath (15) and the first tube (11).

7. The deployment system (10) according to any of the foregoing claims, **characterized in that** the first tube (11) comprises a guidewire lumen (12).

8. The deployment system (10) of any of the foregoing claims, **characterized in that** it further comprises at least one of the following: a first actuating mechanism (16) being linked to the sheath (15) and being slidable in a proximal direction for stepwise retracting the sheath (15); a second actuating mechanism (27) for moving the tip (13) in the distal direction, thus releasing the proximal end (21) of the implant (20); and/or a blocking mechanism being designed such, that a movement of the first actuating mechanism (16) in the proximal direction relative to the tip (13) is limitable to a maximum path of travel by the blocking mechanism (25).

9. The deployment system (10) of claim 8, **characterized in that** the blocking mechanism (25) comprises a punch element (26), spring-loaded wing elements (28), and an abutment element (30).

10. The deployment system (10) of claim 9, **characterized in that** the punch element (26) is forceable in between the resilient wing elements (28), thus forcing the resilient wing elements (28) apart, and into a stressed position abutting against the abutment element (30), thus blocking the first actuating mechanism's (16) path of travel in the proximal direction.

11. The deployment system (10) of claim 9 or 10, **characterized in that** the first actuating mechanism (16) and the abutment element (30) are, prior to the deployment of the vascular implant (20), spaced apart from one another in a distance corresponding to a first retraction length of the sheath (15), the first retraction length of the sheath (15) being such that a medial portion of the implant (20) is released and the proximal (21) end of the implant (20) is covered by the tip and the distal end (22) of the implant (20) is still covered by the sheath (15).

12. The deployment system (10) according to any of claims 8 to 11, **characterized in that** the first actuating mechanism (16) comprises an operator engagable handle having a lever (17) and a grip (18).

13. The deployment system according to any of claims 1 to 12, further comprising a vascular implant (20).

14. The deployment system (10) according to any of claims 1 to 13, wherein the vascular implant (20) is a cardiac valve implant.

## Patentansprüche

1. Freisetzungssystem (10) zur Freisetzung eines Gefäßimplantats, wobei das Freisetzungssystem Folgendes aufweist:
ein erstes Rohr (11), das ein Lumen (12) aufweist und das dazu ausgebildet ist, ein expandierbares Gefäßimplantat auf einem distalen Abschnitt (11a) des ersten Rohrs (11) in einem geladenen Zustand zu tragen,
eine Hülle (15), die dazu ausgebildet ist, um über dem Gefäßimplantat (20) angebracht zu werden, und dieses in einem komprimierten Zustand zu halten, wobei die Hülle (15) relativ zum Gefäßimplantat (20) nach proximal zurückziehbar ist, um das Gefäßimplantat (20) schrittweise freizusetzen und zu expandieren,
ein Eingriffselement (33) zum Eingreifen mit einem distalen Ende (22) des Gefäßimplantants in seinem geladenen Zustand, wobei das Eingriffselement über dem ersten Rohr (11) angeordnet und konzentrisch mit diesem ausgerichtet ist, wobei das Eingriffselement zwischen der Hülle (15) und dem ersten Rohr (11) proximal zum Gefäßimplantat in seinem geladenen Zustand unverschiebbar positioniert ist;
eine Spitze (13), die mit dem ersten Rohr (11) an einem distalen Ende des ersten Rohrs (11) fest verbunden ist, wobei die Spitze (13) ein erstes proximales Ende (13a) und ein zweites distales Ende (13b) aufweist, wobei das erste proximale Ende (13a) einen Anschluss (14) besitzt, um ein proximales Ende (21) des Gefäßimplantats (20) im geladenen Zustand lösbar aufzunehmen und zu halten, wobei die Spitze (13) relativ zum Gefäßimplantat (20) nach distal verschiebbar ist, um das proximale Ende (21) des Gefäßimplantats (20) vom Anschluss (14) freizusetzen,
**dadurch gekennzeichnet, dass** das Freisetzungssystem (10) ferner ein federbelastetes Zapfenelement (29) aufweist, das über dem ersten Rohr (11) verschiebbar angebracht ist und das derart ausgebildet ist, dass das Zapfenelement (29) im geladenen Zustand des Gefäßimplantats (20) an das Eingriffselement (33) anliegt, und dass nach Freisetzen des proximalen Endes (21) des Gefäßimplantats von dem Anschlus (14) der Spitze (13) das Zapfenelement (29) in den Anschluss (14) eingreift und diesen verschließt.

2. Freisetzungssystem nach Anspruch 1, **dadurch gekennzeichnet, dass** das Zapfenelement mit einem Federelement (40) fest verbunden ist.

3. Freisetzungssystem nach Anspruch 2, **dadurch gekennzeichnet, dass** das Federelement (40) eine Druckfeder ist.

4. Freisetzungssystem nach Anspruch 2 oder 3, **dadurch gekennzeichnet, dass** das Federelement (40) einen proximalen Abschnitt (41) ohne Neigungsschräge sowie einen distalen Abschnitt (42) mit Neigungsschräge aufweist.

5. Freisetzungssystem nach einem der Ansprüche 1 bis 4, **dadurch gekennzeichnet, dass** das Zapfenelement (29) ein kompressibles Material aufweist, vorzugsweise beschichtetes oder unbeschichtetes Silikon.

6. Freisetzungssystem (10) nach einem der vorstehenden Ansprüche, **dadurch gekennzeichnet, dass** das Eingriffselement (33) unbeweglich auf einem Schaft (32) getragen ist, welcher über dem ersten Rohr (11) angebracht und mit diesem konzentrisch ausgerichtet ist, wobei der Schaft (32) unverrückbar zwischen der Hülle (15) und dem ersten Rohr (11) positioniert ist.

7. Freisetzungssystem (10) nach einem der vorstehenden Ansprüche, **dadurch gekennzeichnet, dass** das erste Rohr (11) ein Führungsdrahtlumen (12) aufweist.

8. Freisetzungssystem (10) nach einem der vorstehenden Ansprüche, **dadurch gekennzeichnet, dass** es ferner zumindest eines der Folgenden aufweist: einen ersten Betätigungsmechanismus (16), welcher mit der Hülle (15) verbunden ist, und welcher in proximale Richtung verschiebbar ist, um die Hülle (15) schrittweise zurückzuziehen; einen zweiten Betätigungsmechanismus (27), um die Spitze (13) in distale Richtung zu bewegen, um dadurch das proximale Ende (21) des Implantats (20) freizusetzen; und/oder ein Blockierungsmechanismus, der derart ausgebildet ist, dass eine Bewegung des ersten Betätigungsmechanismus (16) in die proximale Richtung relativ zur Spitze (13) durch den Blockierungsmechanismus (25) auf einen maximalen Verschiebeweg begrenzt ist.

9. Freisetzungssystem (10) nach Anspruch 8, **dadurch gekennzeichnet, dass** der Blockierungsmechanismus (25) ein Stempelelement (26), federbelastete Flügelelemente (28) sowie ein Anschlagselement (30) aufweist.

10. Freisetzungssystem (10) nach Anspruch 9, **dadurch gekennzeichnet, dass** das Stempelelement (26) zwischen die nachgiebigen Flügelelemente (28) treibbar ist, wodurch die nachgiebigen Flügelelemente (28) auseinander und in eine belastete Position in Anschlag gegen das Anschlagselement (30) getrieben werden, wodurch der Verschiebeweg des ersten Betätigungsmechanismus (16) in proximale Richtung blockiert wird.

11. Freisetzungssystem (10) nach Anspruch 9, **dadurch gekennzeichnet, dass** der erste Betätigungsmechanismus (16) und das Anschlagselement (30) vor der Freisetzung des Gefäßimplantats (20) räumlich und in einer Distanz voneinander getrennt sind, die einer ersten Rückzugslänge der Hülle (15) entspricht, wobei die erste Rückzugslänge der Hülle (15) derart ist, dass ein mittlerer Abschnitt des Implantats (20) freigesetzt wird und dass das proximale Ende (21) des Implantats (20) durch die Spitze bedeckt wird und das distale Ende (22) des Implantats (20) noch von der Hülle (15) bedeckt ist.

12. Freisetzungssystem (10) nach einem der Ansprüche 8 bis 11, **dadurch gekennzeichnet, dass** der erste Betätigungsmechanismus (16) einen vom Anwender betätigbaren Handgriff mit einem Hebel (17) und einem Griff (18) aufweist.

13. Freisetzungssystem nach einem der Ansprüche 1 bis 12, das ferner ein Gefäßimplantat (20) aufweist.

14. Freisetzungssystem (10) nach einem der Ansprüche 1 bis 13, wobei das Gefäßimplantat (20) ein Herzklappenimplantat ist.

## Revendications

1. Système de pose (10) servant à la pose d'un implant vasculaire, le système de pose comprenant :
un premier tube (11) comprenant une lumière (12) et étant conçu pour transporter un implant vasculaire expansible sur une partie distale (11a) du premier tube (11) dans un état sous sollicitation,
une gaine (15) conçue pour être disposée sur et maintenir l'implant vasculaire (20) dans un état comprimé, la gaine (15) pouvant coulisser dans le sens proximal par rapport à l'implant vasculaire (20) afin de libérer et d'élargir de façon graduelle l'implant vasculaire (20),
un élément d'accouplement (33) destiné à s'accoupler avec une extrémité distale (22) de l'implant vasculaire dans son état sous sollicitation, l'élément d'accouplement étant disposé autour du premier tube (11) et aligné de manière concentrique sur celui-ci, l'élément d'accouplement étant placé d'une manière ne permettant pas un coulissement entre la gaine (15) et le premier tube (11) en position proximale vis-à-vis de l'implant vasculaire dans son état sous sollicitation ;
une pointe (13), qui est raccordée de manière fixe au premier tube (11) au niveau d'une extrémité distale du premier tube (11), la pointe (13) comprenant une première extrémité proximale (13a) et une seconde extrémité distale (13b), la première extrémité proximale (13a) comportant un orifice (14) destiné à recevoir et maintenir de manière détachable une extrémité proximale (21) de l'implant vasculaire (20) dans un état sous sollicitation, la pointe (13) pouvant coulisser dans le sens distal par rapport à l'implant vasculaire (20) afin de libérer l'extrémité proximale (21) de l'implant vasculaire (20) vis-à-vis de l'orifice (14),
**caractérisé en ce que** le système de pose (10) comprend en outre un élément formant obturateur (29) à ressort qui est monté à coulissement sur le premier tube (11) et qui est conçu de telle sorte que, dans l'état sous sollicitation de l'implant vasculaire (20), l'élément formant obturateur (29) soit accolé du côté distal à l'élément d'accouplement (33) et que, à la suite de la libération de l'extrémité proximale (21) de l'implant vasculaire vis-à-vis de l'orifice (14) de la pointe (13), l'élément formant obturateur (29) puisse être accouplé à l'orifice (14) et fermer celui-ci.

2. Système de pose selon la revendication 1, **caractérisé en ce que** l'élément formant obturateur est raccordé de manière fixe à un élément formant ressort (40).

3. Système de pose selon la revendication 2, **caractérisé en ce que** l'élément formant ressort (40) est un ressort de compression.

4. Système de pose selon la revendication 2 ou 3, **caractérisé en ce que** l'élément formant ressort (40) comprend une section à pas ouvert proximale (41) et une section à pas fermé distale (42).

5. Système de pose selon l'une quelconque des revendications 1 à 4, **caractérisé en ce que** l'élément formant obturateur (29) comprend un matériau compressible, de préférence du silicone revêtu ou non revêtu.

6. Système de pose (10) selon l'une quelconque des revendications précédentes, **caractérisé en ce que** l'élément d'accouplement (33) est supporté de manière fixe sur une tige (32) qui est disposée autour du premier tube (11) et alignée de manière concentrique sur celui-ci, la tige (32) étant placée d'une manière ne permettant pas un coulissement entre la gaine (15) et le premier tube (11).

7. Système de pose (10) selon l'une quelconque des revendications précédentes, **caractérisé en ce que** le premier tube (11) comprend une lumière à fil-guide (12).

8. Système de pose (10) selon l'une quelconque des revendications précédentes, **caractérisé en ce qu'**il comprend en outre au moins un des éléments suivants : un premier mécanisme d'actionnement (16) qui est relié à la gaine (15) et qui peut coulisser dans la direction proximale en vue d'une rétraction graduelle de la gaine (15) ; un second mécanisme d'actionnement (27) servant à déplacer la pointe (13) dans la direction distale, de façon à libérer ainsi l'extrémité proximale (21) de l'implant (20) ; et/ou un mécanisme de blocage qui est conçu de telle sorte qu'un déplacement du premier mécanisme d'actionnement (16) dans la direction proximale par rapport à la pointe (13) puisse être limité à un trajet de déplacement maximal par le mécanisme de blocage (25).

9. Système de pose (10) selon la revendication 8, **caractérisé en ce que** le mécanisme de blocage (25) comprend un élément écarteur (26), des éléments formant ailettes (28) à ressort et un élément formant butée (30).

10. Système de pose (10) selon la revendication 9, **caractérisé en ce que** l'élément écarteur (26) peut être poussé de force entre les éléments formant ailettes (28) élastiques, ceci écartant de force les éléments formant ailettes (28) élastiques, et les amenant de force dans une position sous contrainte dans laquelle ils butent contre l'élément formant butée (30), ceci bloquant le trajet de déplacement du premier mécanisme d'actionnement (16) dans la direction proximale.

11. Système de pose (10) selon la revendication 9 ou 10, **caractérisé en ce que** le premier mécanisme d'actionnement (16) et l'élément formant butée (30) sont, avant la pose de l'implant vasculaire (20), espacés l'un de l'autre d'une distance correspondant à une première longueur de rétraction de la gaine (15), la première longueur de rétraction de la gaine (15) étant telle qu'une partie médiane de l'implant (20) soit libérée et que l'extrémité proximale (21) de l'implant (20) soit recouverte par la pointe (13) et que l'extrémité distale (22) de l'implant (20) soit toujours recouverte par la gaine (15).

12. Système de pose (10) selon l'une quelconque des revendications 8 à 11, **caractérisé en ce que** le premier mécanisme d'actionnement (16) comprend un manche manipulable par l'utilisateur comportant un levier (17) et une poignée (18).

13. Système de pose selon l'une quelconque des revendications 1 à 12, comprenant en outre un implant vasculaire (20).

14. Système de pose (10) selon l'une quelconque des revendications 1 à 13, dans lequel l'implant vasculaire (20) est un implant formant valve cardiaque.
